(19) Europäisches Patentamt
European Patent Office
Office européen des brevets

(11) EP 3 321 255 A1

(12) EUROPEAN PATENT APPLICATION

(43) Date of publication:
**16.05.2018 Bulletin 2018/20**

(21) Application number: **17210726.0**

(22) Date of filing: **09.02.2005**

(51) Int Cl.:
***C07D 231/22*** *(2006.01)* ***A61K 31/4152*** *(2006.01)*
***A61P 21/00*** *(2006.01)*

(84) Designated Contracting States:
**AT BE BG CH CY CZ DE DK EE ES FI FR GB GR HU IE IS IT LI LT LU MC NL PL PT RO SE SI SK TR**

(30) Priority: **09.02.2004 JP 2004032420**
**09.02.2004 JP 2004032421**

(62) Document number(s) of the earlier application(s) in accordance with Art. 76 EPC:
**05709984.8 / 1 714 960**

(71) Applicant: **Mitsubishi Tanabe Pharma Corporation**
**Osaka-shi**
**Osaka 541-8505 (JP)**

(72) Inventors:
- **YOSHINO, Hiide**
  **Chiba, 272-0827 (JP)**
- **YONEOKA, Takamoto**
  **Tokyo, 103-8405 (JP)**

(74) Representative: **Ter Meer Steinmeister & Partner**
**Patentanwälte mbB**
**Nymphenburger Straße 4**
**80335 München (DE)**

Remarks:
This application was filed on 27-12-2017 as a divisional application to the application mentioned under INID code 62.

# (54) NOVEL THERAPEUTIC AGENT FOR AMYOTROPHIC LATERAL SCLEROSIS (ALS) OR DISEASE ATTRIBUTABLE TO ALS

(57) There is provided a medicament for treating amyotrophic lateral sclerosis (ALS) or symptoms caused by ALS and/or suppressing the progression thereof, which is characterized in the usage, dosage and administration period of the pyrazolone derivative represented by the following formula (wherein each symbol indicated the same meaning as that defined in the specification):

[Chem. 1]

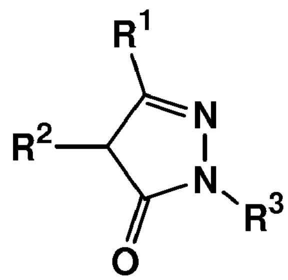

(I)

EP 3 321 255 A1

## Description

TECHNICAL FIELD

The present invention relates to a medicament for treating amyotrophic lateral sclerosis (hereafter sometimes referred to as ALS) or symptoms caused by ALS, and/or suppressing the progression thereof.

ALS which is one of motor neuron diseases is an intractable disease which shows early symptoms such as weakness in the arms, motor dysfunction of the fingers, and fasciculation of the upper extremities, and then shows muscular atrophy, muscular weakness, bulbar palsy, and muscle fasciculation, resulting in respiratory failure. ALS is classified into upper limb type; bulbar type; lower limb type; and a combination thereof, depending on the affected body part. With any type of ALS, body muscle groups of patients become systemically damaged as symptoms progress.

Although the causes of ALS have not yet been revealed sufficiently, major causes of ALS that have been proposed as hypotheses are: (1) autoimmunity (the appearance of autoantibody against Ca channels), (2) excitatory amino acid excess/toxicity (increased extracellular glutamic acid and blocked transport of glutamic acid), (3) oxidative stress disorders (neuronopathy due to abnormality of Cu/Zn superoxide dismutase (SOD) gene and free radical), (4) cytoskeletal disorders (accumulation of neurofilaments in motor nerve cells and appearance of inclusions), and (5) deficiency of neurotrophic factors, and the like.

At present, only riluzole, which blocks glutamatergic neurotransmission in glutamatergic nerves, is an approved pharmaceutical that is effective for suppressing ALS progression. However, there are some reports that the efficacy of riluzole cannot be confirmed. Thus, there has been no consistent evaluation of riluzole.

As described above, ALS is a severe disease that ultimately causes respiratory failure, but there have been no reports of effective medicaments for decreased respiratory function. For instance, it has been known that medicaments such as BDNF (Non-Patent Document 1), RhIGF-1 (Non-Patent Document 2), gabapentin (Non-Patent Documents 3 and 4) and N-acetylcysteine (Non-Patent Document 5), which have been examined as potential medicaments for treating ALS, cannot prevent decreased respiratory function.

Regarding a pyrazolone derivative which is represented by the following formula (I):

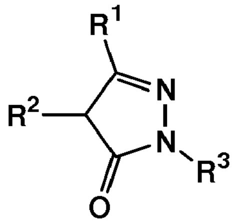

(I)

wherein $R^1$ represents a hydrogen atom, aryl, $C_{1-5}$ alkyl, or $C_{3-6}$ (total carbon number) alkoxycarbonylalkyl, $R^2$ represents a hydrogen atom, aryloxy, arylthio, $C_{1-5}$ alkyl or $C_{1-3}$ hydroxyalkyl, or $R^1$ and $R^2$ are combined with each other to represent $C_{3-5}$ alkylene group, and $R^3$ represents a hydrogen atom, $C_{1-5}$ alkyl, $C_{5-7}$ cycloalkyl, $C_{1-3}$ hydroxyalkyl, benzyl, naphthyl or phenyl, or phenyl substituted with the same or different 1 to 3 substituents selected from the group consisting of $C_{1-5}$ alkoxy, $C_{1-3}$ hydroxyalkyl, $C_{2-5}$ (total carbon number) alkoxycarbonyl, $C_{1-3}$ alkylthio, $C_{1-4}$ alkylamino, $C_{2-8}$ (total carbon number) dialkylamino, halogen atom, trifluoromethyl, carboxyl, cyano, hydroxyl group, nitro, amino and acetamide, examples of the known medical applications include cerebral function-normalizing action (Patent Document 1), lipid peroxide production-suppressing action (Patent Document 1), antiulcer action (Patent Document 2), anti-hyperglycemic action (Patent Document 3), an agent for preventing and treating ophthalmic disease (Patent Document 4), and an agent for treating amyotrophic lateral sclerosis (Patent Document 5).

However, these documents suggest or teach that the aforementioned pyrazolone derivative is useful for ALS treatment, but they do not specifically disclose forms, doses or numbers of doses of the derivative for administration to patients. In addition, it has not been reported that 3-methyl-1-phenyl-2-pyrazoline-5-on is effective for preventing decreased respiratory function in ALS patients.

In general, in order to receive approval to add another efficacy of a pharmaceutical, it is necessary to confirm optimum administration routes of the pharmaceutical in new clinical studies. Further, when administration routes are changed, it is also necessary to examine the possibility of occurrence of adverse effects and the like that have not been previously known. Thus, it is difficult for a person skilled in the art to predict the optimum administration routes of pharmaceuticals.

Non-Patent Document 1: The BDNF study group (Phase III), Neurology, 52, 1427 (1999)
Non-Patent Document 2: Lai EC et al., Neurology, 49, 1621 (1997)

Non-Patent Document 3: Miller RG et al., Neurology 47, 1383 (1996)
Non-Patent Document 4: Miller RG et al., Neurology, 56, 843 (2001)
Non-Patent Document 5: Louwerse ES et al., Arch Neurol., 52, 559 (1995)
Patent Document 1: European Patent Publication EP208874
Patent Document 2: JP Patent Publication (Kokai) No. 3-215425 A (1991)
Patent Document 3: JP Patent Publication (Kokai) No. 3-215426 A (1991)
Patent Document 4: JP Patent Publication (Kokai) No. 7-025765 A (1995)
Patent Document 5: International Publication WO02/34264

DISCLOSURE OF INVENTION

OBJECT TO BE SOLVED BY THE INVENTION

It is an object of the present invention to provide a novel medicament for treating ALS or symptoms caused by ALS and/or suppressing the progression thereof. Further, it is another object of the present invention to provide a method for administering a pyrazolone derivative in a therapeutically effective amount to patients who need ALS treatments while minimizing the occurrence of adverse effect and the like.

MEANS FOR SOLVING THE OBJECT

As a result of intensive studies to attain the above objects, the inventors of the present invention have found that a pharmaceutical composition that has been commercially available as a cerebroprotective agent since June 2001 (generic name: "edaravone;" commercial name: "Radicut;" produced and marketed by Mitsubishi Pharma Corporation) is effective for treating ALS or symptoms caused by ALS and/or suppressing the progression thereof, when its dose regimens, dosages and administration periods are changed. This has led to the completion of the present invention.

BRIEF DESCRIPTION OF THE DRAWINGS

Fig. 1 shows changes in partial pressure of arterial carbon dioxide ($PaCO_2$) of 12 patients for the period from before the 1st administration period until the end of the 6th administration period, during which 2 ampules of "Radicut injection 30 mg" were administered daily in Example 2.

BEST MODE FOR CARRYING OUT THE INVENTION

The present invention relate to the following medicaments.

1. A medicament for treating amyotrophic lateral sclerosis or symptoms caused by amyotrophic lateral sclerosis and/or suppressing the progression thereof, which is administered under the condition that a drug holiday period of 1 day or more is provided once or twice during the period for treating the disease or suppressing the progression of the disease, and which comprises as an active ingredient a pyrazolone derivative represented by the following formula (I) or a physiologically acceptable salt thereof, or a hydrate thereof or a solvate thereof:

[Chem. 2]

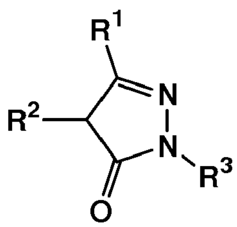

(I)

wherein $R^1$ represents a hydrogen atom, aryl, $C_{1-5}$ alkyl, or $C_{3-6}$ (total carbon number) alkoxycarbonylalkyl, $R^2$ represents a hydrogen atom, aryloxy, arylthio, $C_{1-5}$ alkyl or $C_{1-3}$ hydroxyalkyl, or $R^1$ and $R^2$ are combined with each other to represent $C_{3-5}$ alkylene group, and $R^3$ represents a hydrogen atom, $C_{1-5}$ alkyl, $C_{5-7}$ cycloalkyl, $C_{1-3}$ hydroxyalkyl, benzyl, naphthyl or phenyl, or phenyl substituted with the same or different 1 to 3 substituents selected from the group consisting of $C_{1-5}$ alkoxy, $C_{1-3}$ hydroxyalkyl, $C_{2-5}$ (total carbon number) alkoxycarbonyl, $C_{1-3}$ alkylthio,

$C_{1-4}$ alkylamino, $C_{2-8}$ (total carbon number) dialkylamino, halogen atom, trifluoromethyl, carboxyl, cyano, hydroxyl group, nitro, amino and acetamide.

2. The medicament described in 1, wherein the pyrazolone derivative is 3-methyl-1-phenyl-2-pyrazoline-5-on.

3. The medicament described in 1 or 2, wherein the drug holiday period is provided after a drug administration period of about 7 to 14 days.

4. The medicament described in any one of 1 to 3, wherein a second or subsequent drug administration period is about 5 to 14 days.

5. The medicament described in any one of 1 to 4, wherein the drug holiday period is about 14 to 16 days.

6. The medicament described in any one of 1 to 5, wherein the drug administration period and the drug holiday period are each 14 days.

7. The medicament described in 1 or 2, wherein a course consisting of an initial drug administration period of 14 days and a drug holiday period of 14 days is provided, followed by repetitions of the following combination of periods:

drug administration period: 5 days per week for 2 weeks; and
drug holiday period: 14 days.

8. The medicament described in any one of 1 to 7, wherein the daily dose contains about 15 to 240 mg of a pyrazolone derivative as an active ingredient, or about 15 to 240 mg of a pyrazolone derivative contained in a pharmaceutically acceptable salt of a pyrazolone derivative or a hydrate or solvate of a pyrazolone derivative or a pharmaceutically acceptable salt thereof as an active ingredient.

9. The medicament described in any one of 1 to 8, wherein the daily dose contains about 60 mg of a pyrazolone derivative as an active ingredient, or about 60 mg of a pyrazolone derivative contained in a pharmaceutically acceptable salt of a pyrazolone derivative or a hydrate or solvate of a pyrazolone derivative or a pharmaceutically acceptable salt thereof as an active ingredient.

10. The medicament described in any one of 1 to 9, wherein the administration is carried out once daily.

11. The medicament described in any one of 1 to 10, wherein the administration is a continuous administration.

12. The medicament described in 11, wherein the continuous administration is intravenous infusion administration.

13. The medicament described in 12, wherein the administration rate in the intravenous infusion administration is about 0.5 to 1 mg/minute with respect to a pyrazolone derivative as an active ingredient or a pyrazolone derivative contained in an active ingredient.

14. The medicament described in 11, wherein the continuous administration is an administration form that is substantially equivalent to the intravenous infusion administration wherein the amount of a pyrazolone derivative as an active ingredient or a pyrazolone derivative contained in an active ingredient administered per minute is about 0.5 to 1 mg.

15. The medicament described in any one of 1 to 14 wherein the symptoms caused by amyotrophic lateral sclerosis are decreased respiratory function, voice and speech disorders, dysphagia, or upper and lower extremity motor disorders.

16. The medicament described in any one of 1 to 14 wherein the treatment of amyotrophic lateral sclerosis or symptoms caused by amyotrophic lateral sclerosis and/or the suppression of the progression thereof is a suppression of decrease in respiratory function in amyotrophic lateral sclerosis.

17. A method for administrating as an active ingredient a pyrazolone derivative represented by the following formula (I) or a physiologically acceptable salt thereof, or a hydrate thereof or a solvate thereof, for treating amyotrophic lateral sclerosis or symptoms caused by amyotrophic lateral sclerosis and/or suppressing the progression thereof, wherein a drug holiday period of 1 day or more is provided once or twice during the period for treating the disease or suppressing the progression of the disease,

[Chem. 3]

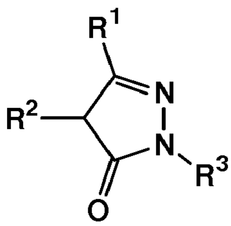

(I)

wherein $R^1$ represents a hydrogen atom, aryl, $C_{1-5}$ alkyl, or $C_{3-6}$ (total carbon number) alkoxycarbonylalkyl, $R^2$ represents a hydrogen atom, aryloxy, arylthio, $C_{1-5}$ alkyl or $C_{1-3}$ hydroxyalkyl, or $R^1$ and $R^2$ are combined with each other to represent $C_{3-5}$ alkylene group, and $R^3$ represents a hydrogen atom, $C_{1-5}$ alkyl, $C_{5-7}$ cycloalkyl, $C_{1-3}$ hydroxyalkyl, benzyl, naphthyl or phenyl, or phenyl substituted with the same or different 1 to 3 substituents selected from the group consisting of $C_{1-5}$ alkoxy, $C_{1-3}$ hydroxyalkyl, $C_{2-5}$ (total carbon number) alkoxycarbonyl, $C_{1-3}$ alkylthio, $C_{1-4}$ alkylamino, $C_{2-8}$ (total carbon number) dialkylamino, halogen atom, trifluoromethyl, carboxyl, cyano, hydroxyl group, nitro, amino and acetamide.

18. The method for administration described in 17, wherein the pyrazolone derivative is 3-methyl-1-phenyl-2-pyrazoline-5-on.

19. The method for administration described in 17 or 18, wherein the drug holiday period is provided after a drug administration period of about 7 to 14 days.

20. The method for administration described in any one of 17 to 19, wherein a second or subsequent drug administration period is about 5 to 14 days.

21. The method for administration described in any one of 17 to 20, wherein the drug holiday period is about 14 to 16 days.

22. The method for administration described in any one of 17 to 21, wherein the drug administration period and the drug holiday period are each 14 days.

23. The method for administration described in 17 or 18, wherein a course consisting of an initial drug administration period of 14 days and a drug holiday period of 14 days is provided, followed by repetitions of the following combination of periods:

drug administration period: 5 days per week for 2 weeks; and
drug holiday period: 14 days.

24. The method for administration described in any one of 17 to 23, wherein the daily dose contains about 15 to 240 mg of a pyrazolone derivative as an active ingredient, or about 15 to 240 mg of a pyrazolone derivative contained in a pharmaceutically acceptable salt of a pyrazolone derivative or a hydrate or solvate of a pyrazolone derivative or a pharmaceutically acceptable salt thereof as an active ingredient.

25. The method for administration described in any one of 17 to 24, wherein the daily dose contains about 60 mg of a pyrazolone derivative as an active ingredient, or about 60 mg of a pyrazolone derivative contained in a pharmaceutically acceptable salt of a pyrazolone derivative or a hydrate or solvate of a pyrazolone derivative or a pharmaceutically acceptable salt thereof as an active ingredient.

26. The method for administration described in any one of 17 to 25, wherein the administration is carried out once daily.

27. The method for administration described in any one of 17 to 26, wherein the administration is a continuous administration.

28. The method for administration described in 27, wherein the continuous administration is intravenous infusion administration.

29. The method for administration described in 28, wherein the administration rate in the intravenous infusion administration is about 0.5 to 1 mg/minute with respect to a pyrazolone derivative as an active ingredient or a pyrazolone derivative contained in an active ingredient.

30. The method for administration described in 27, wherein the continuous administration is an administration form that is substantially equivalent to the intravenous infusion administration wherein the amount of a pyrazolone derivative as an active ingredient or a pyrazolone derivative contained in an active ingredient administered per minute is about 0.5 to 1 mg.

31. The method for administration described in any one of 17 to 30 wherein the symptoms caused by amyotrophic lateral sclerosis are decreased respiratory function, voice and speech disorders, dysphagia, or upper and lower extremity motor disorders.

32. The method for administration described in any one of 17 to 30 wherein the treatment of amyotrophic lateral sclerosis or symptoms caused by amyotrophic lateral sclerosis and/or the suppression of the progression thereof is a suppression of decrease in respiratory function in amyotrophic lateral sclerosis.

The pyrazolone derivative represented by the formula (I) above which is an active ingredient of the present invention, can be synthesized in accordance with any adequate methods. Preferred examples of such methods include a method for producing a pyrazolone derivative of European Patent Publication EP 208874.

As an active ingredient of the present invention, the pyrazolone derivative represented by the formula (I) above in a free form, or any physiologically acceptable salt of the pyrazolone derivative represented by the formula (I) above, or a hydrate thereof or a solvate thereof may be used.

The tautomers (the following formula (I') or (I")) of the pyrazolone derivative exist as described in European

Patent Publication EP 208874. Any of these tautomers may be used as an active ingredient of the medicament of the present invention.

[Chem. 4]

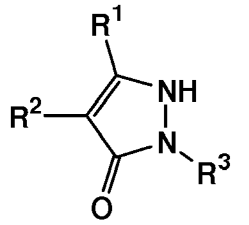

(I')

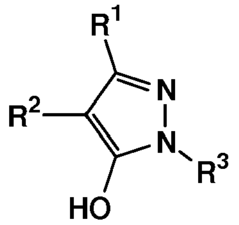

(I'')

In the formula (I), examples of the aryl group in the definition of $R^1$ may include a phenyl group, as well as a phenyl group substituted with a substituent such as a methyl group, a butyl group, a methoxy group, a butoxy group, a chlorine atom or a hydroxy group.

Examples of the $C_{1-5}$ alkyl group in the definition of $R^1$, $R^2$ and $R^3$ may include a methyl group, an ethyl group, a propyl group, an isopropyl group, a butyl group, an isobutyl group, a sec-butyl group, a tert-butyl group, and a pentyl group.

Examples of the $C_{3-6}$ (total carbon number) alkoxycarbonylalkyl group in the definition of $R^1$ include a methoxycarbonylmethyl group, an ethoxycarbonylmethyl group, a propoxycarbonylmethyl group, a methoxycarbonylethyl group and a methoxycarbonylpropyl group.

Examples of the $C_3$-$C_5$ alkylenes in the definitions of $R^1$ and $R^2$ may include a trimethylene group, a tetramethylene group, a pentamethylene group, a hexamethylene group, a methyltrimethylene group, an ethyltrimethylene group, a dimethyltrimethylene group and a methyltetramethylene group.

Examples of the aryloxy group in the definition of $R^2$ may include a phenoxy group, a p-methylphenoxy group, a p-methoxyphenoxy group, a p-chlorophenoxy group and a p-hydroxyphenoxy group. Examples of the arylthio group may include a phenylthio group, a p-methylphenylthio group, a p-methoxyphenylthio group, a p-chlorophenylthio group and a p-hydroxyphenylthio group.

Examples of the $C_3$-$C_5$ alkylenes in $R^1$ and $R^2$ may include a trimethylene group, a tetramethylene group, a pentamethylene group, a methyltrimethylene group, an ethyltrimethylene group, a dimethyltrimethylene group and a methyltetramethylene group.

Examples of the $C_{1-3}$ hydroxyalkyl group in the definition of $R^2$ and $R^3$ may include a hydroxymethyl group, a 2-hydroxyethyl group and a 3-hydroxypropyl group. Examples of the $C_{5-7}$ cycloalkyl group in the definition of $R^3$ may include a cyclopentyl group, a cyclohexyl group and a cycloheptyl group.

In the definition of $R^3$, examples of the $C_{1-5}$ alkoxy group that is the substituent of a phenyl group may include a methoxy group, an ethoxy group, a propoxy group, an isopropoxy group, a butoxy group and a pentyloxy group; examples of the $C_{2-5}$ (total carbon number) alkoxycarbonyl group may include a methoxycarbonyl group, an ethoxycarbonyl group, a propoxycarbonyl group and a butoxycarbonyl group; examples of the $C_{1-3}$ alkylthio group may include a methylthio group, an ethylthio group and a propylthio group; examples of the $C_{1-4}$ alkylamino group may include a methylamino group, an ethylamino group, a propylamino group and a butylamino group; and examples of the $C_{2-8}$ (total carbon number) dialkylamino group may include a dimethylamino group, a diethylamino group, a dipropylamino group, and a dibutylamino group.

Examples of the compound represented by the formula (I) that is used in the present invention may include the following compounds, and a preferred example among them is 3-methyl-1-phenyl-2-pyrazolin-5-one. 3-methyl- 1-phenyl-2-pyrazolin-5-one; 3-methyl-1-(2-methylphenyl)-2-pyrazolin-5-one; 3-methyl-1-(3-methylphenyl)-2-pyrazolin-5-one; 3-methyl-1-(4-methylphenyl)-2-pyrazolin-5-one; 3-methyl-1-(3,4-dimethylphenyl)-2-pyrazolin-5-one; 1-(4-ethylphenyl)-3-methyl-2-pyrazolin-5-one; 3-methyl-1-(4-propylphenyl)-2-pyrazolin-5-one; 1-(4-butylphenyl)-3-methyl-2-pyrazolin-5-one; 1-(3-trifluoromethylphenyl)-3-methyl-2-pyrazolin-5-one; 1-(4-trifluoromethylphenyl)-3-methyl-2-pyrazolin-5- one; 1-(2-methoxyphenyl)-3-methyl-2-pyrazolin-5-one; 1-(3-methoxyphenyl)-3-methyl-2-pyrazolin-5-one; 1-(4-methoxyphenyl)-3-methyl-2-pyrazolin-5-one; 1-(3,4-dimethoxyphenyl)-3-methyl-2-pyrazolin-5-one; 1-(4-ethoxyphenyl)-3-methyl-2-pyrazolin-5-one; 3-methyl-1-(4-propoxyphenyl)-2-pyrazolin-5-one; 1-(4-butoxyphenyl)-3-methyl-2-pyrazolin-5-one; 1-(2-chlorophenyl)-3-methyl-2-pyrazolin-5-one; 1-(3-chlorophenyl)-3-methyl-2-pyrazolin-5-one; 1-(4-chlorophenyl)-3-methyl-2-pyrazolin-5-one; 1-(3,4-dichlorophenyl)-3-methyl-2-pyrazolin-5-one; 1-(4-bromophenyl)-3-methyl-2-pyrazolin-5-one; 1-(4-fluorophenyl)-3-methyl-2-pyrazolin-5-one; 1-(3-chloro-4-methylphenyl)-3-methyl-2-pyrazolin-5-one; 1-(3-methylthiophenyl)-3-methyl-2-pyrazolin-5-one; 1-(4-methylthiophenyl)-3-methyl-2-pyrazolin-5-one; 4-(3-methyl-5-oxo-2-pyrazoline-1-yl) benzoic acid; 1-(4-ethoxycarbonylphenyl)-3-methyl-2-pyrazolin-5-one; 1-(4-nitrophenyl)-3-methyl-2-pyrazolin-5-one; 3-ethyl-1-phenyl-2-pyrazolin-5-one; 1-phenyl-3-propyl-2-pyrazolin-5-one; 1,3-diphenyl-2-pyrazolin-5-one; 3-phenyl-1-(p-tolyl)-2-pyrazolin-5-one; 1-(4-methoxyphenyl)-3-phenyl-2-pyrazolin-5-one; 1-(4-chlorophenyl)-3-phenyl-2-pyrazolin-5-one; 3,4-dimethyl-1-phenyl-2-pyrazolin-5-one; 4-isobutyl-3-methyl-1-phenyl-2-pyrazolin-5-one; 4-(2-hydroxyethyl)-3-methyl-1-phenyl-2-pyrazolin-5-one; 3-methyl-4-phenoxy-1-phenyl-2-pyrazolin-5-one; 3-methyl-4-phenylthio-1-phenyl-2-pyrazolin-5-one; 2,3a, 4,5,6,7-hexahydro-2-phenylindazole-3-one; 3-(ethoxycarbonylmethyl)-1-phenyl-2-pyrazolin-5-one; 1-phenyl-2-pyrazolin-5-one; 3-methyl-2-pyrazolin--5-one; 1,3-dimethyl-2-pyrazolin-5-one; 1-ethyl-3-methyl-2-pyrazolin-5-one; 1-butyl-3-methyl-2-pyrazolin-5-one; 1-(2-hydroxyethyl)-3-methyl-2-pyrazolin-5-one; 1-cyclohexyl-3-methyl-2-pyrazolin-5-one; 1-benzyl-3-methyl-2-pyrazolin-5-one; 1-(a-naphthyl)-3-methyl-2-pyrazolin-5-one; 1-methyl-3-phenyl-2-pyrazolin-5-one; 3-methyl-1-(4-methylphenyl)-2-pyrazolin-5-one; 1-(4-butylphenyl)-3-methyl-2-pyrazolin-5-one; 1-(4-methoxyphenyl)-3-methyl-2-pyrazolin-5-one; 1-(4-butoxyphenyl)-3-methyl-2-pyrazolin-5-one; 1-(4-chlorophenyl)-3-methyl-2-pyrazolin-5-one; 1-(4-hydroxyphenyl)-3-methyl-2-pyrazolin-5-one; 1-(3,4-dihydroxyphenyl)-3-methyl-2-pyrazolin-5-one; 1-(2-hydroxyphenyl)-3-methyl-2-pyrazolin-5-one; 1-(3-hydroxyphenyl)-3-methyl-2-pyrazolin-5-one; 1-(4-hydroxyphenyl)-3-methyl-2-pyrazolin-5-one; 1-(3,4-hydroxyphenyl)-3-methyl-2-pyrazolin-5-one; 1-(4-hydroxyphenyl)-3-phenyl-2-pyrazolin-5-one; 1-(4-hydroxymethylphenyl)-3-methyl-2-pyrazolin-5-one; 1-(4-aminophenyl)-3-methyl-2-pyrazolin-5-one; 1-(4-methylaminophenyl)-3-methyl-2-pyrazolin-5-one; 1-(4-ethylaminophenyl)-3-methyl-2-pyrazolin-5-one; 1-(4-butylaminophenyl)-3-methyl-2-pyrazolin-5-one; 1-(4-dimethylaminophenyl)-3-methyl-2-pyrazolin-5-one; 1-(acetamidophenyl)-3-methyl-2-pyrazolin-5-one; and 1-(4-cyanophenyl)-3-methyl-2-pyrazolin-5-one:

As the salt of the pyrazolone derivative of formula (I) above, acid addition salts and base addition salts can be used. Examples thereof may include mineral acid salts such as hydrochloride, sulfate, hydrobromide, and phosphate; organic acid salts such as methanesulfonate, para-toluenesulfonate, acetate, oxalate, citrate, malate, and fumarate; metal salts such as sodium salt, potassium salt, and magnesium salt; ammonium salts; and organic amine salts such as ethanolamine and 2-amino-2-methyl-1-propanol. However, examples thereof are not particularly limited thereto as long as a physiologically acceptable salt is used.

One or more types of the compound represented by the formula (I) or salts thereof, which is an active ingredient of the medicament of the present invention, can be directly administered to a patient, and preferably, should be administered as a preparation in the form of a pharmaceutical composition containing the active ingredient and pharmacologically and pharmaceutically acceptable additives, which is well-known to a person skilled in the art.

Examples of pharmacologically and pharmaceutically acceptable additives may include excipients, disintegrators or adjuvants for the disintegrators, binders, lubricants, coating agents, dye, diluents, bases, resolvents or solubilizers, isotonizing agents, pH modifiers, stabilizers, propellants, and adhesives. Examples of preparations, which are appropriate for oral administration, can include tablets, capsules, powders, fine granules, granules, solutions, and syrups. Examples of preparations, which are appropriate for parenteral administration, can include injections, drops, adhesive preparation, and suppositories.

Examples of additives for preparations which are appropriate for oral administration may include: excipients such as glucose, lactose, D-mannitol, starch or crystalline cellulose; disintegrators or adjuvants for disintegrators, such as carboxymethylcellulose, starch or carboxymethylcellulose calcium; binders such as hydroxypropylcellulose, hydroxypropylmethylcellulose, polyvinylpirrolidone, or gelatin; lubricants such as magnesium stearate, or talc; coating agents such as hydroxypropylmethylcellulose, saccharose, polyethyleneglycol, or titanium oxide; and bases such as petrolatum, liquid paraffin, polyethylene glycol, gelatin, kaolin, glycerine, purified water, or hard fat.

Examples of additives for preparations that can be appropriately used for injection or drip include a resolvent or a solubilizer that can compose an aqueous injection, such as distilled water for injection, physiological saline, and propylene glycol, or an injection to be dissolved before use; isotonizing agents such as glucose, sodium chloride, D-mannitol, and glycerine; and pH modifiers such as inorganic acid, organic acid, inorganic base or organic base.

A protective agent for the brain (drops) comprising as an active ingredient a compound represented by the above formula (I) has already been clinically used (under the general name "edaravone" and the commercial name "Radicut": produced and marketed by Mitsubishi Pharma Corporation). This commercially available pharmaceutical preparation can be used as it is as the pyrazolone derivative which is used for the medicament and the administration method according to the present invention.

In general, when a human body is found to have a disease, appropriate treatments are provided by physicians. An example of such a treatment is medicament therapy. In general medicament therapy practice, continuous drug administration is carried out until the disease is cured. In contrast, in accordance with the medicament and the method for administering the same according to the present invention, a drug holiday period of 1 day or more is provided once or twice during medicament therapy. That is, the present invention is characterized in that a course consisting of a drug administration period and a drug holiday period is considered to be a unit, and that the unit is repeated at least twice. Here, when such unit is repeated at least twice, it always ends with a drug holiday period. However, it is not essential

to provide a drug holiday period at the end. Specifically, in a case where a course consists of two repetitions of such unit, the course is carried out in the following order: "a drug administration period, a drug holiday period, a drug administration period, and a drug holiday period." In such case, the present invention also encompasses a course of "a drug administration period, a drug holiday period, and a drug administration period," in which a drug holiday period is not provided at the end. In the present invention, the term "drug holiday period" indicates a period in which drug administration is not carried.

Durations of a drug administration period and a drug holiday period are not particularly limited, as long as each period lasts 1 day or more. Such durations can be appropriately selected based on observation of a patient. The durations of these periods may be the same or different. Further, durations of an initial drug administration period and an initial drug holiday period and durations of a second or subsequent drug administration period and a second or subsequent drug holiday period may be the same or different. For instance, it is possible to provide a course consisting of a 1-day drug administration, a 1-day drug holiday, a 1-day drug administration, and a 1-day drug holiday. It is also possible to provide a course consisting of a 1-day drug administration, a 2-day drug holiday, a 3-day drug administration, and a 4-day drug holiday.

Here, the number of days required for an initial drug administration period is, for example, preferably about 1 to 14 days. Specific examples thereof are 1, 2, 5, 7, 10, and 14 days, preferably 1, 2, 5, 7, and 14 days, more preferably 7 and 14 days, and further preferably 14 days. The number of days required for a second or subsequent drug administration period is, for example, preferably about 1 to 14 days. Specific examples thereof are 1, 2, 5, 7, 10, and 14 days, preferably 1, 2, 5, 7, and 14 days, more preferably 5 days and 14 days, and further preferably 14 days. The number of days required for a drug holiday period is, for example, preferably about 1 to 16 days. Specifically, examples thereof are 1, 2, 7, 9, 14, and 16 days, preferably 2, 14, and 16 days, more preferably 14 and 16 days, and further preferably 14 days. A particularly preferred example of a course of a drug administration period and a drug holiday period consists of an initial drug administration period of 14 days and a drug holiday period of 14 days, followed by repetitions of the following combination of periods:

drug administration period: 5 days per week for 2 weeks; and
drug holiday period: 14 days.

The daily dose of an active ingredient can be appropriately determined depending on conditions such as a patient's age and physical condition. In general, the daily dose of free form of pyrazolone derivative for adults (an amount of a pyrazolone derivative as an active ingredient, or an amount of a pyrazolone derivative contained in an active ingredient that is a physiologically acceptable salt of a pyrazolone derivative or a hydrate or solvate of a pyrazolone derivative or a physiologically acceptable salt thereof) is preferably about 15 to 240 mg, more preferably about 30 to 60 mg, and further preferably about 60 mg.

Numbers of administration per day during a drug administration period are not limited, and they can be appropriately determined based on observation of patient conditions. However, considering the burden placed upon a patient and the like, the active ingredient is preferably administered once, twice, or three times, and more preferably once daily.

When administering the active ingredient, administration routes are not particularly limited. The active ingredient can be orally or parenterally administered. Further, bolus administration and continuous administration are possible. Continuous administration is preferred. Examples of continuous administration may include intravenous infusion administration, transdermal administration, oral administration using sublingual tablets, and oral or intrarectal administration using sustained-release formulations. Intravenous infusion administration is preferred. When bolus administration by injection or intravenous infusion administration is carried out, injection products such as that described in JP Patent Publication (Kokai) No. 63-132833 A (1988) are preferably used.

Upon intravenous infusion administration, the dosing rate of a free form of pyrazolone derivative is preferably about 0.5 to 1 mg/minute. Based on such dosing rate, the duration for administration is determined to be about 15 to 480 minutes, preferably about 30 to 120 minutes, more preferably about 30 to 60 minutes, and further preferably about 60 minutes.

Administration that may be carried out is substantially equivalent in terms of pharmacokinetics to intravenous infusion administration wherein an amount of a free form of pyrazolone derivative administered per minute is about 0.5 to 1 mg. Specifically, when such administration is carried out, the serum concentration time course of an unchanged free form of pyrazolone derivative of the administered pyrazolone derivative (including a physiologically acceptable salt thereof, a hydrate or solvate thereof) becomes substantially equivalent to that obtained via the aforementioned intravenous infusion administration. Examples thereof include transdermal administration, oral administration using sublingual tablets, and oral or intrarectal administration using sustained-release formulations.

Examples of symptoms caused by ALS may include decreased respiratory function, voice and speech disorders, dysphagia, and upper and lower extremity motor disorders. In the present invention, decreased respiratory function is a preferred example. The term "symptoms caused by ALS" should be as broadly interpreted as possible in conformity

with the above definition. Thus, it should not be interpreted differently depending on differences in disease names. A skilled physician can readily diagnose whether or not a disease corresponds to ALS.

In addition, examples of treatment of ALS or symptoms caused by ALS and/or suppression of progression thereof may include suppression of decrease in respiratory function in amyotrophic lateral sclerosis.

EXAMPLES

The present invention is more specifically described by the Examples below, but the scope of the present invention is not limited to the following Examples.

Example 1: Efficacy evaluation 6 months after administration based on ALSFRS-R (Revised ALS functional rating scale)(reference: *"Noshinkei"* (Cerebral Nerve) 53 (4): 346-355, 2001)

(30 mg group)

1 ampule of "Radicut injection 30 mg" (containing 30 mg of edaravone, produced and marketed by Mitsubishi Pharma Corporation) was intravenously administered once daily to 5 patients of ALS. Single daily administration of the medicament took 30 minutes, and the administration was carried out for 14 consecutive days (the 1st administration period). After the 1st administration period, patients were observed for two weeks (a drug holiday period). Thereafter, intravenous administration of the medicament was carried out for 10 days (with no administration on Saturdays, Sundays, and national holidays) in the manner described above (the 2nd administration period). Then, treatments similar to those carried out in the 2nd administration period were repeated 4 times (the 3rd to 6th administration periods).

(60 mg group)

2 ampules of the aforementioned "Radicut injection 30 mg" were intravenously administered once daily to 14 patients of ALS. Single daily administration of the medicament took 60 minutes, and the administration was carried out for 14 consecutive days (the 1st administration period). After the 1st administration period, patients were observed for two weeks (a drug holiday period). Thereafter, the intravenous administration of the medicament was carried out for 10 days (with no administration on Saturdays, Sundays, and national holidays) in the manner described above (the 2nd administration period). Then, treatments similar to those carried out in the 2nd administration period were repeated 4 times (the 3rd to 6th administration periods).

<ALSFRS-R>

a) Evaluation method based on cumulative differences

In accordance with ALSFRS-R, the score at a time point "before the 1st administration period" (*) was determined to be a base point based on comparison of changes "before administration" and changes "during administration." Then, patients were evaluated in the following manner.

[Table 1]

Evaluation schedule of ALSFRS-R

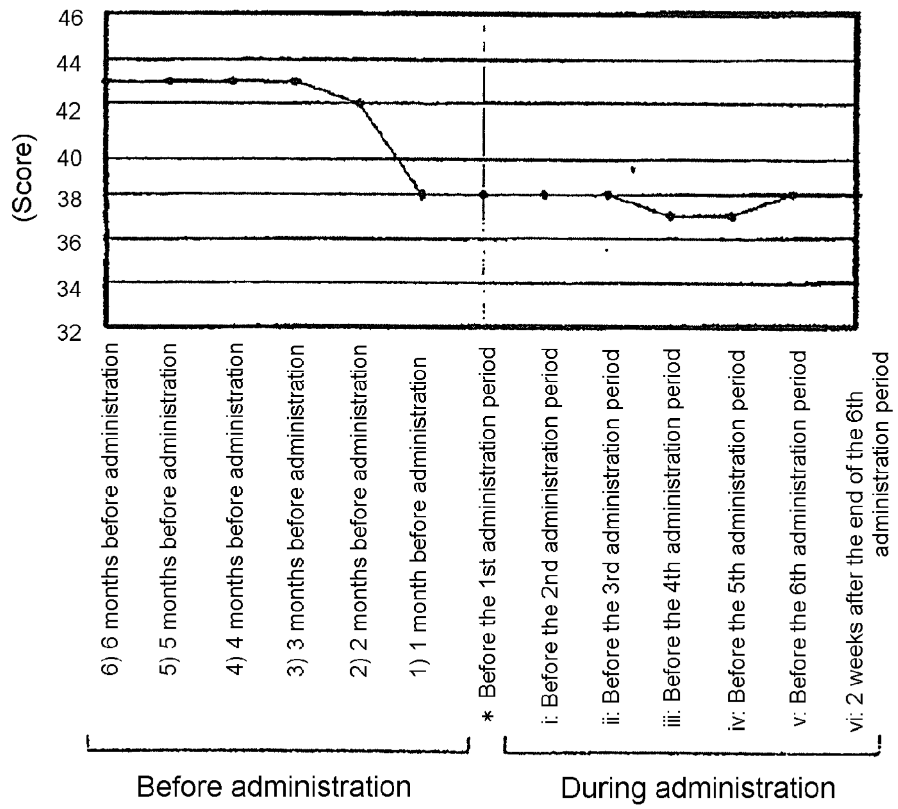


1) Obtainment of differences

Difference at each time point before administration = (* before the 1st administration period) - (time point 1, 2 3, 4, 5 or 6 before administration) Difference at each time point during administration = (time point i, ii, iii, iv, v, or vi before each administration period) - (* before the 1st administration period)

2) Obtainment of the sum of differences obtained in 1)

The sum of differences at time points before administration (defined as A)

$$A = (* - 1) + (* - 2) + (* - 3) + (* - 4) + (* - 5) + (* - 6)$$

The sum of differences at time points during administration (defined as B)

$$B = (i - *) + (ii - *) + (iii - *) + (iv - *) + (v - *) + (vi - *)$$

3) Obtainment of the average of differences

Averages of the sums of differences obtained in 2) were calculated so as to obtain cumulative differences. (The sums were divided by 6, based on 6 time points 1 to 6 before administration and 6 time points i to vi during administration).

$$\text{Cumulative difference before administration} = A / 6$$

$$\text{Cumulative difference during administration} = B / 6$$

4) Obtainment of ratio between the cumulative differences

Based on the averages obtained in 3), the ratio of cumulative difference before administration (A / 6) to cumulative difference during administration (B / 6) was expressed by the following formula:

(Formula) cumulative difference during administration / cumulative difference before administration × 100.

That is, the ratio was determined by the following equation:

$$\text{Ratio between cumulative differences (\%)} = (B / 6) / (A / 6) \times 100.$$

5) Criteria

Based on the "ratio between cumulative differences" obtained in 4), efficacy evaluation was made in accordance with the following criteria. The ratios of 50% or less was determined to be "suppressed", the ratios of more than 50% and less than 100% was determined to be "slightly suppressed", and the ratios of 100% or more were determined to be "no change".

b) Assessment based on cumulative differences

Table 2 shows results of assessment based on cumulative differences of individual cases of each drug administration group in accordance with ALSFRS-R. The suppression rates (rates of "suppressed") were 20% (1 out of 5 cases) in the 30 mg group, and 50% (7 out of 14 cases) in the 60 mg group.

[Table 2]

| Assessment based on cumulative differences in accordance with ALSFRS-R | | | | |
|---|---|---|---|---|
| Drug administration group | Assessment | | | |
| | Suppressed | Slightly suppressed | No change | Suppression rate |
| 30 mg group | 1 | 3 | 1 | 20.0% |
| 60 mg group | 7 | 1 | 6 | 50.0% |

The aforementioned results clearly indicate that the medicament and the method for administering the same of the present invention exert effects of suppressing a decrease in the ALSFRS-R score which is a rating scale for amyotrophic lateral sclerosis.

Example 2: Efficacy evaluation 6 months after administration based on %FVC and $PaCO_2$

The term "%FVC" stands for percent predicted forced vital capacity; it is generally used as an index in a method for objectively evaluating respiratory function in ALS patients (ALS treatment guidelines 2002). In addition, in the case of ALS patients (placebo group), the rate of decrease of %FVC for during 6 months is 13.8% (The BDNF Study Group (Phase III), Neurology, 52, 1427 (1999)).

(30 mg group)

1 ampule of the aforementioned "Radicut injection 30 mg" was intravenously administered once daily to 4 patients of ALS. Single daily administration of the medicament took 30 minutes, and it was carried out for 14 consecutive days (the $1^{st}$ administration period). After the $1^{st}$ administration period, patients were observed for two weeks (a drug holiday period). Thereafter, intravenous administration of the medicament was carried out for 10 days (with no administration on Saturdays, Sundays, and national holidays) in the manner described above (the $2^{nd}$ administration period). Then, treatments similar to those carried out in the $2^{nd}$ administration period were repeated 4 times (the $3^{rd}$ to the $6^{th}$ administration periods).

The %FVC for each patient was determined using a chestac-11 (Chest). The average rate of decrease of %FVC was 9.3% when comparing %FVC before the 1st drug administration period and that at the end of the 6th drug administration period.

(60 mg group)

2 ampules of "Radicut injection 30 mg" (each of which contained 30 mg of edaravone) were intravenously administered once daily to 12 patients of ALS. Single daily administration of the medicament took 60 minutes and it was carried out for 14 consecutive days (the 1st administration period). After the 1st administration period, patients were observed for two weeks (a drug holiday period). Thereafter, intravenous administration of the medicament was carried out for 10 days (with no administration on Saturdays, Sundays, and national holidays) in the manner described above (the 2nd administration period). Then, treatments similar to those carried out in the 2nd administration period were repeated 4 times (the 3rd to the 6th administration periods).

The %FVC for each patient was determined using a chestac-11 (Chest), as with the case of the 30 mg group. The average rate of decrease of %FVC was 4.5% when comparing %FVC before the 1st drug administration period and that at the end of the 6th drug administration period. In addition, partial pressure of arterial carbon dioxide ($PaCO_2$) was determined. The $PaCO_2$ of each patient was determined using a blood gas analyzer (Bayer 850; Bayer Medical). $PaCO_2$ values before the 1st administration period and those after the end of the 6th administration period were almost the same. Fig. 1 shows changes in $PaCO_2$ values for 12 patients during the period from before the 1st administration period to after the end of the 6th administration period.

From the above results, it is understood that administration of "Radicut injection 30 mg" significantly suppressed both the decrease of %FVC and the increase of $PaCO_2$ in ALS patients, and that respiratory function was maintained.

Example 3: Safety evaluation 6 months after administration

The patients of Example 2 were each subjected to a clinical laboratory test.

(Determination method)

The following components were determined before and after drug administration using a large automated multichannel analyzer (automatic analyzer 7600-020s; Hitachi). As is apparent from the values (average values) before and after drug administration which were listed below, values after edaravone administration via the method for administering the medicament of the present invention did not increase to abnormal levels. Thus, the present invention was found to have no problem with respect to safety.

[Table 3]

| Examined component | Before administration | After administration |
|---|---|---|
| GOT (IU/L) | 21.3 | 19.1 |
| GPT (IU/L) | 22.2 | 19.6 |
| γ-GTP (IU/L) | 31.6 | 25.3 |
| BUN (mg/dl) | 14.1 | 14.3 |
| Creatinine (mg/dl) | 0.6 | 0.6 |
| CK (IU/L) | 165.3 | 135.6 |

Industrial Applicability

The medicament and the method for administering the same of the present invention are effective for treating ALS or symptoms caused by ALS and/or suppressing the progression thereof. With use of the medicament and the method for administering the same of the present invention, it is possible to reduce numbers of doses or clinic visits. Thus, patients are less constrained by hospitalization so that the burdens placed upon patients can be reduced.

The present application is based on Japanese Patent Application Nos. 2004-032420 and 2004-032421 filed on February 9, 2004. These applications are incorporated herein by reference in their entirety.

## Claims

1. 3-Methyl-1-phenyl-2-pyrazoline-5-on or a physiologically acceptable salt thereof, or a hydrate thereof or a solvate thereof for use in the treatment of amyotrophic lateral sclerosis or symptoms caused by amyotrophic lateral sclerosis and/or suppressing the progression thereof, wherein a course consisting of an initial drug administration period of 14 days and a drug holiday period of 14 days is provided during the period for treating the disease or suppressing the progression of the disease, followed by repetitions of the following combination of periods:

   drug administration period: 10 days; and
   drug holiday period: 14 days.

[Fig. 1]

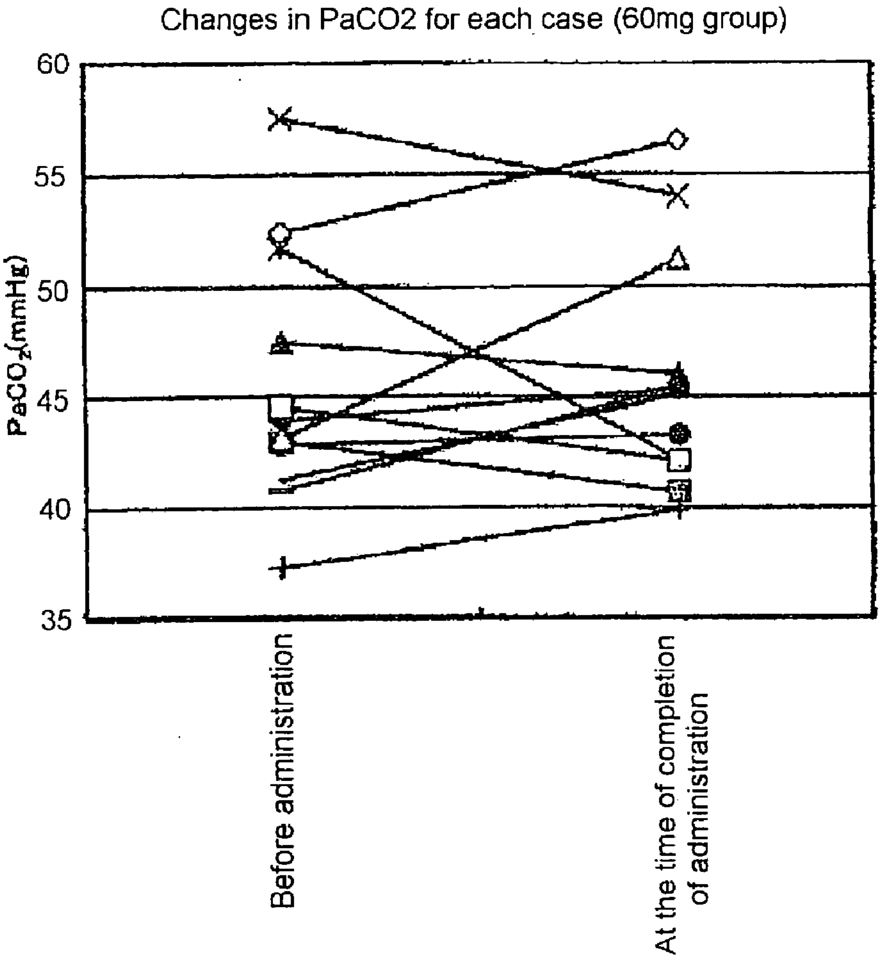
Changes in PaCO2 for each case (60mg group)
60
55
50
45
40
35
$PaCO_2$(mmHg)
Before administration
At the time of completion of administration

Europäisches Patentamt
European Patent Office
Office européen des brevets

# EUROPEAN SEARCH REPORT

Application Number
EP 17 21 0726

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 02/34264 A1 (MITSUBISHI PHARMA CORP [JP]; IKEDA KEN [JP]) 2 May 2002 (2002-05-02) * column 7 - paragraph 36 * * claims 1,4,9,14 * & EP 1 405 637 A1 (MITSUBISHI PHARMA CORP [JP]) 7 April 2004 (2004-04-07) * column 7 - paragraph 36 * * claims 1,4,9,14 * ----- | 1 | INV. C07D231/22 A61K31/4152 A61P21/00 |
| A | YAMAMOTO T ET AL: "Delayed neuronal death prevented by inhibition of increased hydroxyl radical formation in a transient cerebral ischemia", BRAIN RESEARCH, ELSEVIER, AMSTERDAM, NL LNKD- DOI:10.1016/S0006-8993(97)00490-3, vol. 762, no. 1-2, 1 January 1997 (1997-01-01), pages 241-242, XP002278046, ISSN: 0006-8993 * the whole document * ----- | 1 | |

TECHNICAL FIELDS SEARCHED (IPC)

A61K
A61P

1

The present search report has been drawn up for all claims

| Place of search | Date of completion of the search | Examiner |
|---|---|---|
| Munich | 21 March 2018 | Werner, Doris |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 21 0726

This annex lists the patent family members relating to the patent documents cited in the above-mentioned European search report. The members are as contained in the European Patent Office EDP file on
The European Patent Office is in no way liable for these particulars which are merely given for the purpose of information.

21-03-2018

| Patent document cited in search report | Publication date | Patent family member(s) | Publication date |
|---|---|---|---|
| WO 0234264 A1 | 02-05-2002 | AT 303808 T | 15-09-2005 |
| | | DE 60022560 D1 | 13-10-2005 |
| | | DE 60022560 T2 | 29-06-2006 |
| | | EP 1405637 A1 | 07-04-2004 |
| | | ES 2248144 T3 | 16-03-2006 |
| | | JP 3758164 B2 | 22-03-2006 |
| | | JP WO2002034264 A1 | 04-03-2004 |
| | | US 6933310 B1 | 23-08-2005 |
| | | WO 0234264 A1 | 02-05-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 208874 A
- JP 3215425 A
- JP 3215426 A
- JP 7025765 A
- WO 0234264 A
- JP 63132833 A
- JP 2004032420 A
- JP 2004032421 A


### Non-patent literature cited in the description


- The BDNF study group (Phase III). *Neurology,* 1999, vol. 52, 1427
- **LAI EC et al.** *Neurology,* 1997, vol. 49, 1621
- **MILLER RG et al.** *Neurology,* 1996, vol. 47, 1383
- **MILLER RG et al.** *Neurology,* 2001, vol. 56, 843
- **LOUWERSE ES et al.** *Arch Neurol.,* 1995, vol. 52, 559
- The BDNF Study Group (Phase III). *Neurology,* 1999, vol. 52, 1427